# EUROPEAN PATENT APPLICATION

(11) **EP 3 771 908 A1**
(43) Date of publication of application: **03.02.2021**
(21) Application number: 19382648.4
(22) Date of filing: 29.07.2019
(51) Int. Cl.: G01N 33/558, G01N 33/543, G01N 27/447

(54) **LATERAL-ELECTROPHORETIC BIOASSAY**

(71) Applicant: Fundació Institut Català de Nanociència i Nanotecnologia (ICN2), 08193 Bellaterra (ES)
(72) Inventor: SENA-TORRALBA, Amadeo, E-46970 Alaquàs, València (ES); ÁLVAREZ, Ruslan, E-08031 Barcelona (ES); MERKOÇI HYKA, Arben, E-08290 Cerdanyola del Vallés, Barcelona (ES)
(74) Representative: ABG Intellectual Property Law, S.L.

(57) **Abstract**

The present invention relates to a modified lateral flow test device together with methods for its use in the point-of-care detection and quantification of one or more analytes of interest within a sample, wherein the sample moves across the paper strip by means of electrophoresis

## Description

### TECHNICAL FIELD OF THE INVENTION

The present invention relates to a test strip device and a method suitable for the on-site detection of an analyte in a sample. This invention finds practical application in the medical field (e.g. *in vitro* prognosis, diagnosis and follow-up evaluation) as well as in a number of other industries including veterinary medicine, quality control, product safety in food production, and environmental health and safety.

### BACKGROUND OF THE INVENTION

Devices that can perform fast analysis and diagnostics near the patient are known as bedside or point-of-care (PoC) tests. Point-of-care diagnostics are an attractive alternative to the well-established benchtop tests, which are expensive, time consuming, and requires qualified personnel. While limitations regarding sensitivity, selectivity and versatility have delayed them from becoming part of the routine medical testing, daily, PoC devices are evolving into important tools for diagnostics, which are required not only by medical specialists at hospitals or the doctor's office but anyone, even at home or outdoors.

PoC examples include, but are not limited to, blood glucose testing, metabolic testing (e.g., thyroid stimulating hormone), blood gas and electrolytes analysis, rapid coagulation testing, rapid cardiac markers diagnostics, drugs of abuse screening, urine testing, pregnancy testing, fecal occult blood analysis, food pathogen screening, hemoglobin diagnostics, infectious disease testing, cholesterol screening, cancer testing (e.g. PSA), hormone testing (hCG, LH, FSH), cardiac (troponin), pulmonary, gastroenterology (e.g., H. pylori antibodies), urology, nephrology, dermatology, neurology, pediatrics, surgical, and public health (Ebola, cholera, HIV, malaria), and combinations thereof.

One testing method that is often employed for point-of-care testing and more conventional testing involves the use of a lateral flow assay. The lateral flow assay (LFA) (also known as strip test) is a paper-based platform for the detection, and in certain cases quantification, of target analytes in liquid samples (matrix), where the sample is placed on a test device and the results are rapidly displayed. Low development costs and ease of production of LFAs have resulted in the expansion of its applications to multiple fields in which rapid tests are required. LFA-based tests are widely used in hospitals, physician's offices and clinical laboratories for the qualitative and quantitative detection of specific antigens and antibodies, as well as products of gene amplification. A variety of biological samples can be tested using LFAs, including urine, saliva, sweat, serum, plasma, whole blood and other fluids. Further industries in which LFA-based tests are employed include veterinary medicine, quality control, product safety in food production, and environmental health and safety. In these areas of utilization, rapid tests are used to screen for animal diseases, pathogens, chemicals, toxins and water pollutants, among others.

LFA methods and devices have been described extensively. See, e.g., EP 0306772 A1, EP 0349215 A1, WO 90/06511 A1, EP 0987551 A2, EP 1197754 A1, WO 2007/055712 A1, WO 2009/003177 A1, WO 2013/158504 A1, WO 2013/158505 A1, WO 2015/138558 A1, WO 2015/150067 A1, WO 2016/020790 A1, WO 2016/022647 A1, WO 2016/022655 A1, WO 2016/022872 A1, EP 3076177 A1, EP 3128324 A1, EP 3153858 A1, EP 3306320 A1. Such assays involve the detection of an analyte substance, often generally called ligand, that has specific binding with a receptor substance that is present in the test device. Thus, the receptor is capable of distinguishing a specific ligand or ligands from other sample constituents having similar characteristics. Immunological assays involving reactions between antibodies and antigens are one such example of a specific binding assay [then, the test may be referred to as lateral flow immunoassay (LFIA)]. Other examples include DNA and RNA hybridization reactions and binding reactions involving hormones and other biological receptors. One well-known commercial embodiment of this technique is the Clearblue One-Step Pregnancy Test.

The simplest LFA is a dipstick, which is dipped into the sample solution. Besides the dipstick format, LFAs exist in which the strip is inserted into a rigid plastic cassette provided with a sample well and normally a reading window. More recently the development in this field has been extended toward the microfluidic paper analytical devices (µPADs), which are characterized by the use of patterning to create flow channels (i.e. a directed flow). All these configurations may be collectively referred to as LFA.

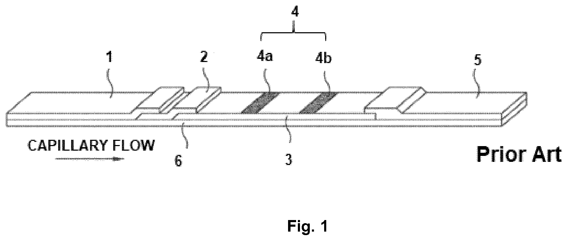

Although there are a number of different variations of the technology, they all operate using the same basic concept. The principle behind the LFA is simple: a liquid sample (or its extract) containing the analyte of interest moves without the assistance of external forces (i.e. by capillary action) through a polymeric strip, which is normally divided in various zones that are generally assembled on a baking material (6) for better stability and handling and on which molecules that can interact with the analyte are attached. Strips used for LFA generally contain four main components: (i) sample application pad (1); (ii) conjugate pad (2); (iii) membrane (3) with a detection zone (4), normally further divided into test and control lines (4a,4b); and (iv) absorbent pad (5). A typical lateral flow assay format is shown in Figure 1. The sample to be tested is applied at one end of the strip, onto the sample application pad (1). The liquid fraction of the sample then moves through a conjugate release pad (2) onto which a conjugate has been dried. In the so-called "sandwich" set-up, the conjugate consists of detection molecules specifically directed against the analyte of interest and indicator particles, such as colloidal gold and latex microspheres. Upon contact with the liquid sample, the conjugate redissolves and specifically binds to any analyte present in the sample to form an analyte-conjugate complex. This complex flows through a membrane (3), normally made of nitrocellulose, on which test and control reagents have been immobilized in the detection area (4). More specifically, detection area (4) is normally provided with two capture lines, or regions, arranged sequentially and positioned perpendicularly to the flow direction, each containing bound reagents. Test line (4a) contains analyte-specific molecules which are able to bind to and immobilize the analyte-conjugate complex, resulting in a visible colored line (or otherwise detectable). Control line (4b) does not contain analyte-specific molecules but is able to fix non-bound conjugate-containing particles. The formation of a colored line at control line (4b) indicates that the test sample has flowed past test line (4a). The color intensity observed at test line (4a) is directly proportional to the analyte concentration in the sample and therefore enables semi-quantitative interpretation of the test result. If the analyte of interest is present at a level above the detection limit, test line (4a) and control line (4b) both become clearly visible. If the analyte is present at a level below the detection limit, only control line (4b) becomes visible during the test. The read-out, represented by the lines appearing with different intensities, can be assessed by eye or using a dedicated reader. In order to test multiple analytes simultaneously under the same conditions, additional test lines of capture molecules specific to different analytes can be immobilized in an array format. On the other hand, multiple test lines loaded with the same capture molecules can be used for semi-quantitative assays. The principle of this 'ladder bars' assay is based on the stepwise capture of colorimetric conjugate-analyte complexes by the capture immobilized molecules on each successive line, where the number of lines appearing on the strip is directly proportional to the concentration of the analyte. The last component of the rapid test device is an absorbent pad (5) (also known as a wicking or sink pad) which collects the fluid flowing through the test system and prevents any backflow of fluid. Absorbent pad (5) allows the use of samples whose volume exceeds the wicking capacity of nitrocellulose membrane (3).

LFAs have a number of desirable characteristics including their ease of use and broad applicability to a variety of analytes. However, LFAs are suffering a delay in their transition from laboratory to clinic mainly due to the hindrance to detect biomarkers in a complex sample medium. As noted above, lateral flow assay is performed in a porous membrane, usually nitrocellulose or another paper substrate, in which the movement of the sample takes place by capillarity. When working with complex samples, such as serum or whole blood, the viscosity blocks the nitrocellulose membrane and opaque matrix components hinder the optical signal in the detection zone reducing the test usability.

Despite the fact that many publications regarding the application of lateral flow assay for diagnosis in whole blood or serum has been reported, all of them require several preliminary steps such as diluting of the sample or its pre-treatment with trichloro-acetic acid (TCA) or tween-20. A few other researchers have tried to overcome the LFA limitation when dealing with opaque media e.g., whole blood by changing from optical to magnetic or electrical detections.

Moreover, while currently available LFAs are particularly well-suited for determining whether or not one or more test substances are present in a sample above a given detection limit, they are poorly suited for providing quantitative results. There is an ongoing need in the art for devices and methods that combine the ease of use characteristics of lateral flow tests with systems that are designed to provide quantitative results. Such devices and methods may, for example, allow medical practitioners, and even patients, to diagnose and monitor a variety of conditions at the point of care (e.g., chair-side, at home, or essentially anywhere in the world) without being tied to a medical facility or a testing laboratory.

There is thus still a need for portable, simple and easy-to-use devices and methods able to conduct detection of analytes in complex real samples. Preferably these tests should also allow the determination of quantitative results in a straightforward and reliable manner.

### BRIEF DESCRIPTION OF THE INVENTION

The present invention solves the aforementioned needs by the provision of a PoC test suitable for separating and detecting target analytes in complex media, providing accurate quantitative results, if needed.

The present inventors have conducted extensive experimentation with an intention to provide a novel portable assay that overcomes the limitation of high matrix effect associated with traditional LFA biosensors. Among other approaches, the use of electrophoresis was envisaged by the inventors as an integrated purification strategy with the aim of improving the detection and quantification of analytes. Gel electrophoresis has been widely used for many years in molecular biology laboratories with different purposes such as separation of DNA fragments in DNA fingerprinting, analysing the polymerase chain reaction results and also to study the structure of proteins. Basically, electrophoresis requires an electric potential difference and a detection system. The system consists of anode and cathode electrodes connected by an electrolyte solution. The electrophoretic velocity (*v*) of the charged particles depends on the particles mobility (m) and the field strength (E), being the mobility determined by the particle's size, shape and charge. Moreover, the electrical parameters such as current, voltage, power and the factors such as ionic strength, pH value, viscosity and pore size determine the electrophoresis conditions. Previously, portable electrophoresis devices have been developed for biosensing applications, although not as a purification but as a detection method through the concept of moving boundary reaction. Systems such as gel electrophoresis or highly advance automated microchip electrophoresis are known to provide a great degree of resolution and they are among the electrophoretic techniques of choice at the time for separating molecules. However, the inventors found that such techniques were unsuitable for the purposes of the present invention *inter alia* because highly demanding electrical parameters and/or costly or complex equipment are required.

After extensive research, the present inventors have unexpectedly found that paper electrophoresis, a technique that has been superseded as a method for separation by more modern and advance technologies, may be successfully applied and have developed a paper-based electrophoretic strip test intended for point-of-care use, herein also referred to as Lateral-Electrophoretic Bioassay (LEB), that is able to achieve efficient separation and detection of analytes in complex real samples such as serum. Thus, the invention surprisingly brings together separation (taking advantages of paper electrophoresis) and detection (through LFA) in a simple and easy to use device. Moreover, paper substrates are cheap, abundant, recyclable and biosustainable.

Advantageously, the device may be coupled to a smartphone or any other portable computing device used as optical readout and/or as a power source capable of generating up to 240 v. Successful separation of several compounds has been carried out in less than 15 minutes and LEB sensing capabilities have been tested with real samples such as serum. To the best of the inventors this is the first time that an immunosensor can detect in one single step an analyte in such complex matrix.

In an aspect, the present invention relates to a strip test device comprising:
- means for supporting one or more paper strips for lateral flow assay, said paper strips comprising a sample application area (1) and a detection area (4) comprising capture elements; and
- means for producing an electrical potential difference in said one or more paper strips between the sample application area (1) and the detection area (4).

In another aspect the present invention relates to a method for the detection of an analyte in a sample with the device of the invention, the method comprising:
- dispensing the sample onto the application area (1) of the one or more paper strips, wherein said sample either has been previously treated with conjugate outside the device or is treated with conjugate present on the device;
- producing an electrical potential difference across the one or more paper strips so that the sample starts migrating along the one or more paper strips by electrophoresis; and
- continuing the migration of the sample towards the detection area (4) of the one or more paper strips so that the presence of analyte may be determined by a detectable signal at the detection area (4).

In a particular embodiment, the method is also suitable for the quantification of an analyte in a sample (test sample), said method further comprising obtaining a calibration curve from calibration samples containing known or allocated amounts of analyte and quantifying the analyte in the test sample using the calibration curve and the value of the signal generated with the test sample. The invention can include quantifying the target analyte in the test sample algebraically using signals generated by one or more calibration samples.

The point-of-care strip test device disclosed herein may be employed for instance to detect the presence of an analyte that is indicative of a disorder or condition such as infectious diseases, pregnancy, microbial infections, cancer, autoimmune disorders, cardiac disorders, allergic disorders, drug abuse, and the like. Thus, prognosis and/or initial diagnosis and/or treatment or post-treatment monitoring and/or decision-making may be based on the test results.

Further aspects of the invention relate to kits comprising the device of the invention, instructions for use of the device and optionally one or more paper strips.

These aspects and preferred embodiments thereof are additionally also defined hereinafter in the detailed description and in the claims.

### BRIEF DESCRIPTION OF THE FIGURES

To better understand the invention, its objects and advantages, the following figures are attached to the specification in which the following is depicted:
**Figure 1****:** Scheme of a conventional LFA, wherein the flow is generated by capillarity, comprising: a receiving pad (1) on which the sample is applied; a conjugate area (2) on which dissolvable conjugates are stored; a reaction or analytical membrane (3) with a detection area (4) comprising a test line (4a) and a control line (4b); an absorbent pad (5); a backing material (6). In some cases a cover tape (not shown) is also used on the assay strip to prevent fast solvent evaporation and hold the elements in place.

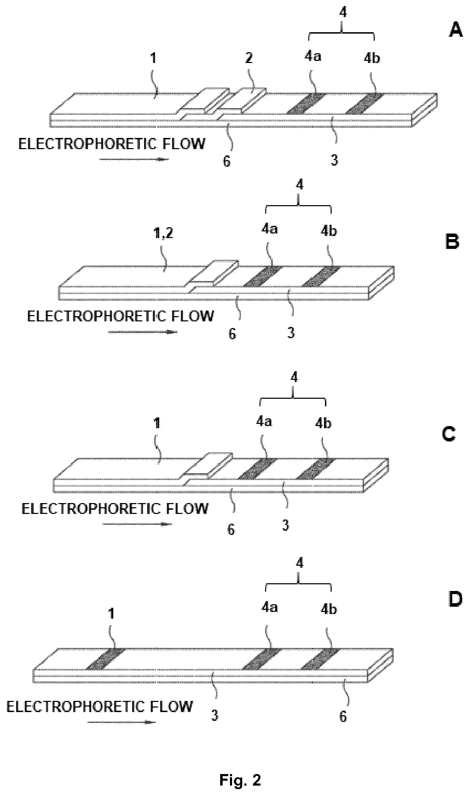

**Figure 2****:** Schemes of the Lateral-Electrophoretic Bioassay (LEB) according to different embodiments of the present invention, wherein the flow is generated by electrophoresis. **A**) The LEB comprises: a receiving pad (1) on which the sample is applied; a conjugate area (2) on which dissolvable conjugates are stored; a reaction or analytical membrane (3) with a detection area (4) comprising a test line (4a) and a control line (4b); a backing material (6). **B**) The LEB comprises: a pad (1,2) for receiving the sample with conjugate storage means; a reaction or analytical membrane (3) with a detection area (4) comprising a test line (4a) and a control line (4b); a backing material (6). **C**) The LEB comprises: a receiving pad (1) on which a sample pre-mixed with conjugate is applied; a reaction or analytical membrane (3) with a detection area (4) comprising a test line (4a) and a control line (4b); a backing material (6). **D**) The LEB comprises: a membrane (3) comprising a sample application area (1) on which a sample pre-mixed with conjugate is applied; a detection area (4) comprising a test line (4a) and a control line (4b); a backing material (6). The backing material (6) as well as any cover tape or cassette (not shown) are optional elements.

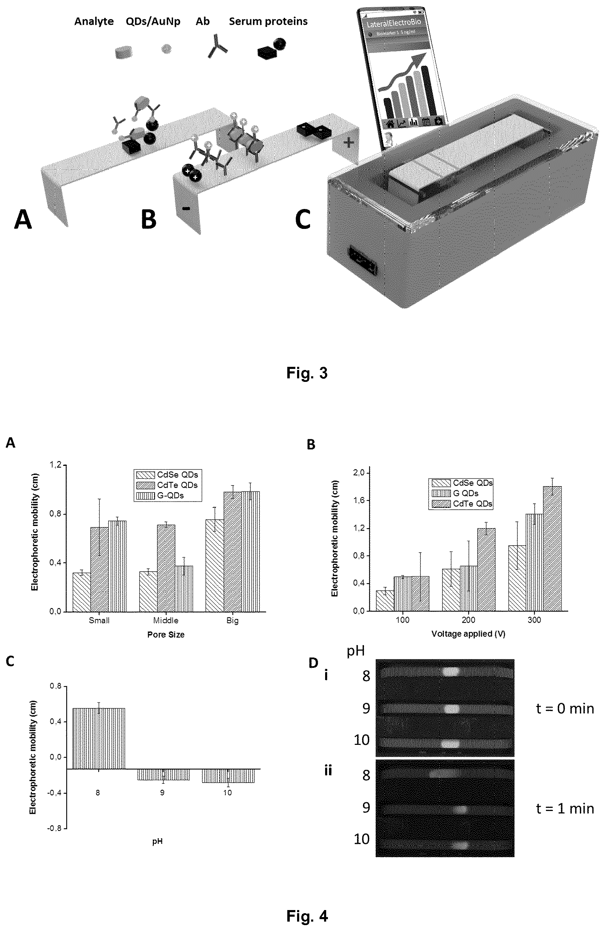

**Figure 3****:** Scheme of the Lateral-Electrophoretic Bioassay (LEB) according to the examples disclosed herein. **A**) The bioconjugated QDs are incubated with the sample and the mixture is drop-casted onto the nitrocellulose strip. The sample contains the target analyte and a high amount of interfering components that will mask the assay signal. **B**) By applying electrophoresis on the paper strip, the components in the sample will be forced to move and due to their difference in surface charge, the target analyte will be purified from the interfering serum proteins and will be detected by the capture antibodies. **C**) The bioassay is carried out in a point-of-care device operated via smartphone.
**Figure 4****:** Electrophoresis performance evaluation according to example 1. **A**) The effect of the nitrocellulose membrane pore size on the electrophoretic mobility of different QDs. **B**) Evaluation of the voltage effect applied on the electrophoresis mobility using the same QDs. **C**) Effect of the pH value on the electrophoretic mobility. **D**) GQDs image at different pH values before applying the voltage 0 min (i) and after 1 min of voltage (ii).

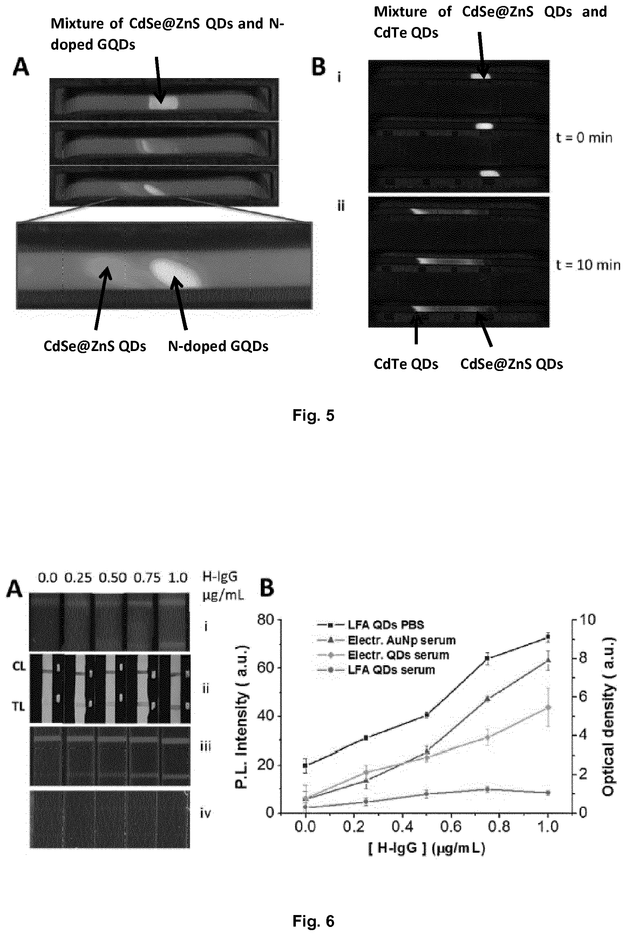

**Figure 5****:** Separation of QDs by electrophoresis according to example 1. **A**) N-S-doped G-QDs are mixed with CdSe QDs and drop-casted in the centre of the nitrocellulose membrane strip that is immersed in electrophoresis buffer pH 8. After running the electrophoresis for 10 minutes, the CdSe QDs (pink) moves to the left side of the strip (negative electrode) separating from the N-S-doped G-QDs (blue), which have slightly moved to the left side. **B**) **i** CdTe QDs are mixed with CdSe QDs and drop-casted in the centre of the nitrocellulose membrane strip that is immersed in electrophoresis buffer pH 8. ii After running the electrophoresis for 10 minutes, the CdTe QDs (orange) have moved faster to the left side (negative electrode) separating from the CdSe QDs, which have slightly moved to the left side.
**Figure 6****:** Detection of H-IgG in human serum according to example 1. **A**) Picture of the nitrocellulose membrane strips after performing the i) Electrophoresis assay in serum using QDs ii) Electrophoresis assay in PBS using AuNps iii) Lateral flow assay in PBS using QDs (prior art; comparative) iv) Lateral flow assay in serum using QDs (prior art; comparative). (camera parameters ISO 160, A 1/10, Manual focus). **B**) Calibration curve showing the normalized values of signal intensity in the TL for the four detection methods with increasing concentrations of H-lgG.

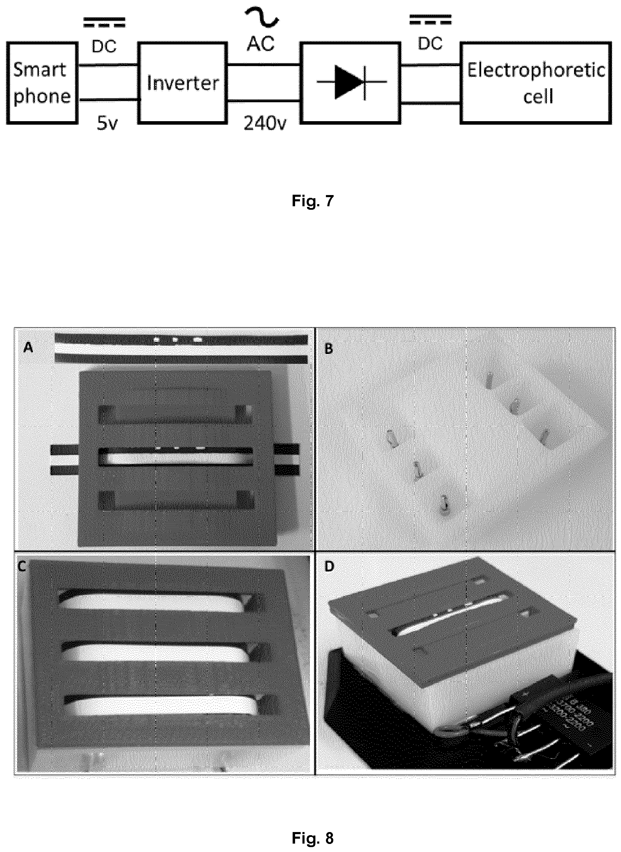

**Figure 7****:** Diagram of the components and connections in the electrophoretic device according to a preferred embodiment of the invention.
**Figure 8****:** Images of the Lateral-Electrophoretic Bioassay (LEB) device according to example 1. **A**) The nitrocellulose membrane strip is delimited with wax and inserted in a 3D printed support that keeps it straight and stretched. **B**) The electrophoresis is performed with 6 reservoirs made of Teflon containing the electrodes. **C**) The reservoirs are filled with electrophoresis buffer and the end of three nitrocellulose membrane strips are submerged in the reservoirs. The buffer flows by capillarity along the nitrocellulose membrane strip acting as a bridge between the reservoirs. **D**) The 6 buffer reservoirs are attached on a 3D printed platform with the electronic components.

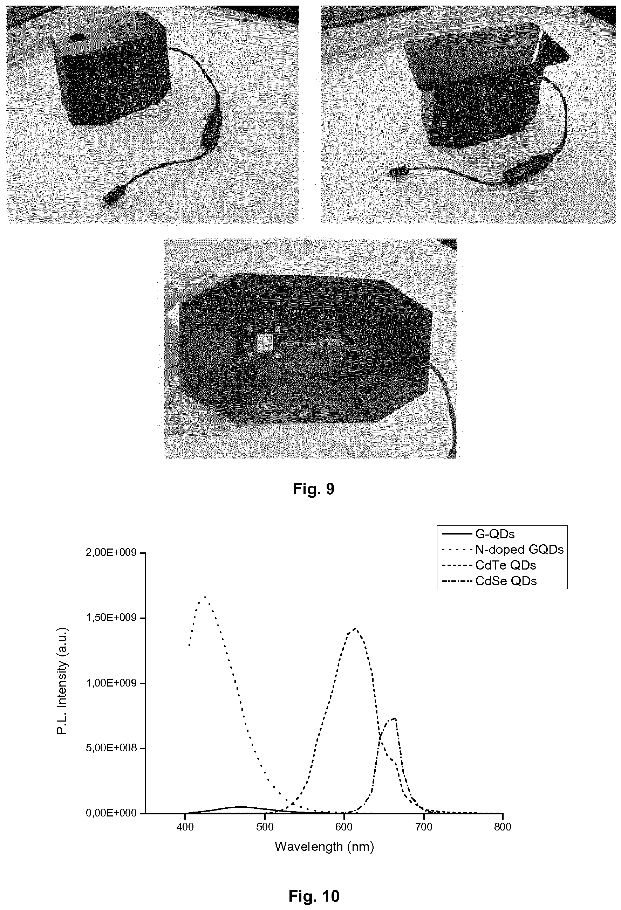

**Figure 9****:** Pictures of the cover of the device containing the LEDs according to example 1.
**Figure 10****:** Wavelength emission spectrum in example 1 of the N-S-doped G-QDs (dotted line), G-QDs (solid line), CdTe QDs (dashed line) and CdSe@ZnS QDs (dashdotted line).

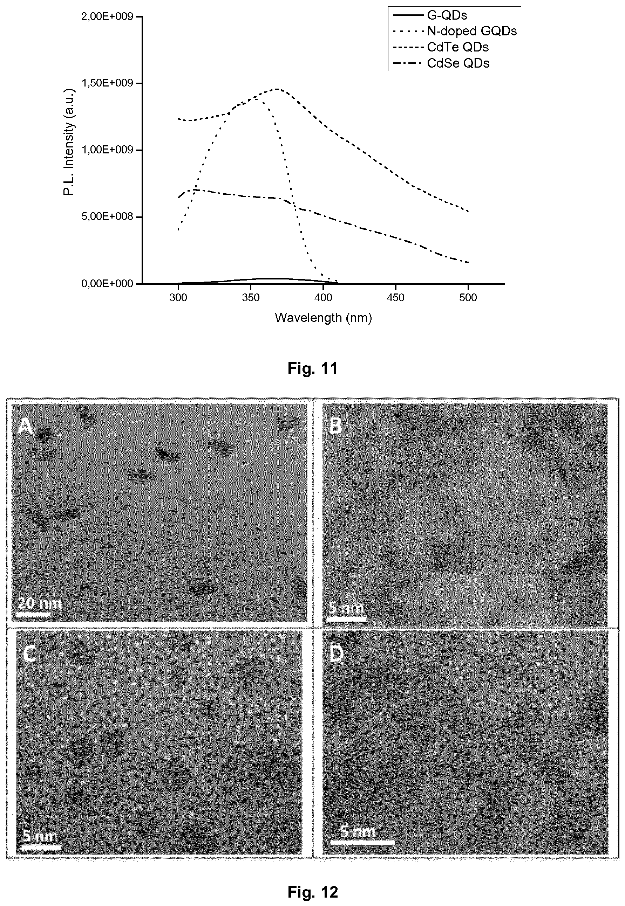

**Figure 11****:** Wavelength excitation spectrum in example 1 of the N-S-doped G-QDs (dotted line), G-QDs (solid line), CdTe QDs (dashed line) and CdSe@ZnS QDs (dashdotted line).
**Figure 12****:** TEM images in example 1 of (A) CdSe@ZnS QDs (B) CdTe QDs (C) G-QDs (D) N-S-doped G-QDs.

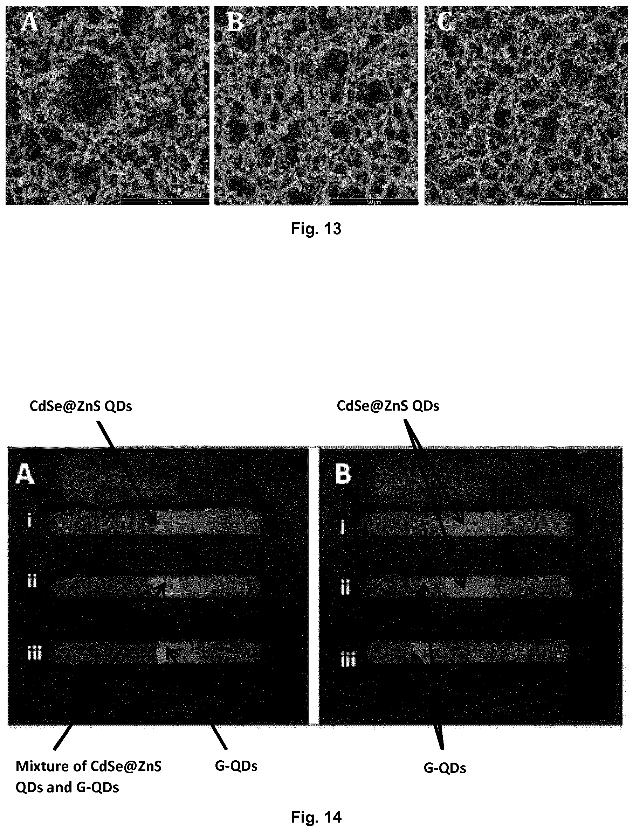

**Figure 13****:** SEM images in example 1 of (A) HF075 (B) HF170 (C) HF240 nitrocellulose membranes.
**Figure 14****:** Electrophoresis of CdSe@ZnS and G-QDs in example 1. A) Picture of strips at 0 minutes, before starting the electrophoresis for i) CdSe QDs ii) Mixture of CdSe QDs and G-QDs and iii) G-QDs. B) Picture of strips after 10 minutes of electrophoresis for i) CdSe QDs ii) Mixture of CdSe QDs and GQDs and iii) G-QDs.

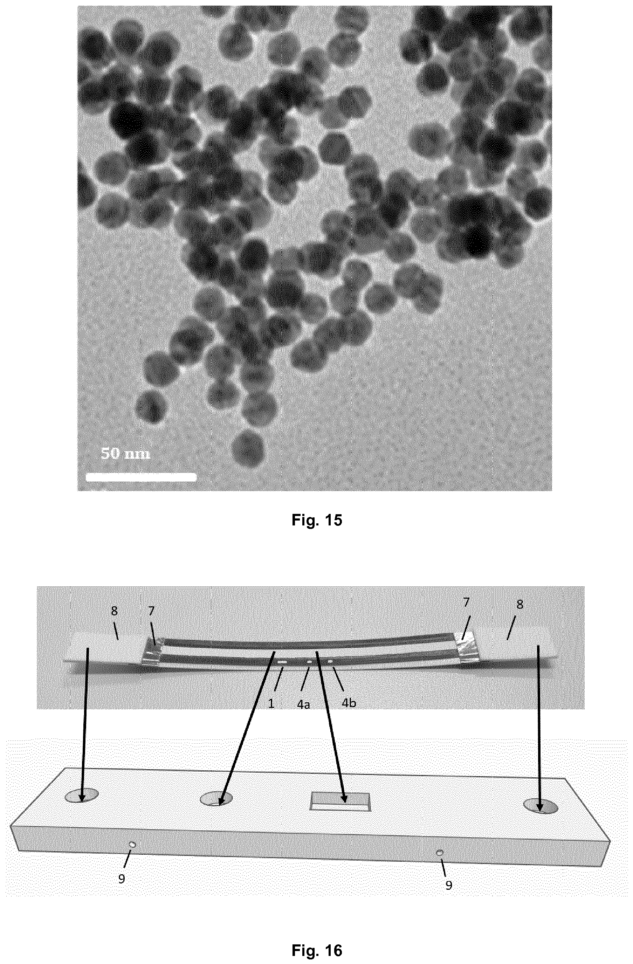

**Figure 15****:** TEM image of the AuNps suspension in example 1.
**Figure 16****:** Device of the Lateral-Electrophoretic Bioassay (LEB) according to example 2. The device uses a wax-patterned strip comprising sample application area (1), test line (4a), control line (4b), two electrodes (7) and two cellulose pads (8) and the strip is housed in a cassette comprising orifices for applying the buffer onto the cellulose pads (8), applying the sample onto the sample application area (1) and for the electrode connections (9) as well as a reading window for visualizing the test and control lines (4a, 4b) in the detection area (4).

### DETAILED DESCRIPTION OF THE INVENTION

Unless defined otherwise, all technical and scientific terms and expressions used herein have the same meaning as commonly understood to one of ordinary skill in the art to which this disclosure belongs.

The present invention relates to devices and methods for determining the presence or amount of an analyte substance in a sample by means of one or more specific binding reactions. More particularly, the present invention provides a modified lateral flow test system together with methods for its use in the point-of-care (on-site) detection and quantification of one or more analytes of interest within a sample, such as a biological sample.

The strip test device of the present invention comprises:
- means for supporting one or more paper strips intended or suitable for lateral flow assay onto which a sample to be tested is applied; and
- electrophoretic means for making the sample move through the strips.

More particularly, the device comprises means for supporting (e.g. holding or fixing) at least one paper-based strip for lateral flow assay comprising a sample application area (1) and a detection area (4) comprising capture elements, wherein the device is provided with an electrophoretic system to make the sample migrate from the application area (1) to the detection area (4) of the strip when a potential is applied.

Advantageously, the present invention allows running electrophoretic separation and detection through LFA in a single step.

Thus, the device of the invention employs conventional lateral flow assay strips comprising: a sample application region (1), optionally a conjugate region (2) and a detection region (4). These strips are commercially available for a wide range of analysis and are normally disposable. Materials and reagents generally employed in lateral flow test devices, and well known to those of skill in the art, are contemplated for the present invention. However, unlike traditional lateral flow assays currently available, the device disclosed herein does not rely on capillary action to move the sample across the strip but on electrophoresis. In consequence, no wicking pad is necessary in the strips to be used in the present invention.

According to one embodiment, the format of the device is selected from a dipstick (to be dipped into the sample solution) or as rigid plastic cassette provided with a sample well. Further, the device of the invention may be a microfluidic paper analytical device (µPAD), that is, wherein the paper strip is patterned to generate microfluidic channels.

The term "point of care" as used herein is primarily intended to refer to devices, methods and the like that are suitable for on-site analysis of samples, i.e. outside the laboratory, in a reliable manner and preferably in a short period of time (e.g. less than 30 min). Examples described herein are testing methods and devices that can be used to provide rapid, accurate, affordable laboratory-quality quantitative testing at the point of care. Such devices are designed to eliminate or replace expensive, centralized laboratory testing equipment and technical personnel. Such devices may include automated data reporting and decision support. The point of care testing as provided herein is highly applicable in all fields of science and has many uses in human and veterinary medicine, quality control, food and feed analysis and environmental and water analysis. In medicine, point of care refers to the analysis of a sample on or near a site of patient care, including but not limited to hospitals, emergency departments, intensive care units, primary care setting, medical centers, patient homes, a physician's office, a pharmacy or a site of an emergency. The point of care testing as provided herein is accomplished through the use of portable or handheld instruments which may be designed for an easy-to-use operation. The goal being to collect the patient sample and obtain the analytical data in a (relatively) short period of time at or (relatively) near the location of the patient.

As used herein, the term "analyte" generally refers to a substance to be detected. In certain instances, the term "analyte" may be used as a synonym of the term "marker" or "biomarker". For instance, analytes may include antigenic substances, haptens, antibodies, and combinations thereof. Analytes also include, but are not limited to, toxins, organic compounds, proteins, peptides, microorganisms, amino acids, nucleic acids, hormones, steroids, vitamins, drugs (including those administered for therapeutic purposes as well as those administered for illicit purposes), drug intermediaries or byproducts, bacteria, virus particles and metabolites of or antibodies to any of the above substances. In one embodiment, the analyte is selected from the group consisting of an agrochemical, a pesticide residue, a diagnostic marker protein, a hormone, a drug, and a drug residue. In some examples, an antigen present in a biological sample may serve as an analyte, wherein the antigen binds to an antibody immobilized on a substrate during analysis. Specific examples of some analytes include ferritin; creatinine kinase MB (CK-MB); digoxin; phenyloin; phenobarbitol; carbamazepine; vancomycin; gentamycin; theophylline; valproic acid; quinidine; luteinizing hormone (LH); follicle stimulating hormone (FSH); estradiol, progesterone; C-reactive protein; lipocalins; IgE antibodies; cytokines; vitamin B2 micro-globulin; glycated hemoglobin (Gly. Hb); cortisol; digitoxin; N-acetylprocainamide (NAPA); procainamide; antibodies to rubella, such as rubella-IgG and rubella IgM; antibodies to toxoplasmosis, such as toxoplasmosis IgG (Toxo-IgG) and toxoplasmosis IgM (Toxo-IgM); testosterone; salicylates; acetaminophen; hepatitis B virus surface antigen (HBsAg); antibodies to hepatitis B core antigen, such as anti-hepatitis B core antigen IgG and IgM (Anti-HBC); human immune deficiency virus 1 and 2 (HIV 1 and 2); human T-cell leukemia virus 1 and 2 (HTLV); hepatitis B e antigen (HBeAg); antibodies to hepatitis B e antigen (Anti-HBe); influenza virus; thyroid stimulating hormone (TSH); thyroxine (T4); total triiodothyronine (Total T3); free triiodothyronine (Free T3); carcinoembryoic antigen (CEA); lipoproteins, cholesterol, and triglycerides; and alpha fetoprotein (AFP). Drugs of abuse and controlled substances include, but are not intended to be limited to, amphetamine; methamphetamine; barbiturates, such as amobarbital, secobarbital, pentobarbital, phenobarbital, and barbital; benzodiazepines, such as librium and valium; cannabinoids, such as hashish and marijuana; cocaine; fentanyl; LSD; methaqualone; opiates, such as heroin, morphine, codeine, hydromorphone, hydrocodone, methadone, oxycodone, oxymorphone and opium; phencyclidine; and propoxyhene.

The term "sample" as used herein refers to a volume of a liquid, solution or suspension, intended to be subjected to qualitative or quantitative determination of any of its properties, such as the presence or absence of a component, the concentration of a component, etc. As used herein, the term "sample" includes both biological and non-biological samples. The term "biological sample" includes, but is not limited to, urine, saliva, sweat, serum, plasma, blood, lymph, mucus, feces, other bodily secretions, cells, and tissue sections obtained from a human or non-human organism. A buffer comprising one or more biomolecules may also be considered as a biological sample. In other instances, the sample can be related to food testing (e.g. milk, water), environmental testing (e.g. plant and soil extract, water), bio-threat or bio-hazard testing, etc.

The test strip provides a lateral flow path for liquids. The membrane and other components of the test strip are said to be in "fluid communication" if liquid, such as a liquid sample or buffer, can flow from one component to another. If components of the test strip are in contact with each other, they are in fluid communication. However, as will be recognized by one of skill in the art, direct contact between two particular components is not required for fluid communication.

The sample application area (1) is the region onto which can be placed a liquid test sample suspected of containing an analyte.

The sample application area (1), and optionally a conjugate area (2), may form part of a membrane (3) comprising the detection area (4); figure 2D illustrates an embodiment of this configuration. Alternatively, the sample application area (1) and the optional conjugate area (2) may not form part of the membrane (3) comprising the detection area (4) but be present as the so-called "pads"; figures 2A, 2B and 2C illustrate embodiments of this configuration.

The sample application area (1) may thus be formed by or comprise a sample pad (also known as sample receiving pad or simply receiving pad). Sample pads employed in a conventional lateral flow assay device may be used in the present invention. This is an absorbent pad on to which the sample is applied. Materials useful for the sample pad include, but are not limited to, woven mesh, cellulose, cotton linter, glass fiber, rayon, polypropylene, and/or other commonly used filtration media known in the art and at the discretion of the artisan. In a particular embodiment, the sample pad is made of cellulose.

As shown in figure 2A, the strip may comprise a sample pad (1), then optionally a conjugate pad (2), and finally a membrane (3) comprising a detection area (4). The contact between all of these components, normally by partial overlapping, in combination with the electrophoretic system allows the sample to move through the test strip when a potential is applied.

In a preferred embodiment, the sample application area (1) is located before the conjugate area (2) and is in communication with said conjugate area, more preferably, the sample pad (1) is overlying partially or totally the conjugate area (2) or is overlaid partially or totally by the conjugate area (2). In a further embodiment the sample pad (1) is mounted on the conjugate pad (2).

According to another configuration, the sample pad for receiving the sample (1) may comprise conjugate means (2), as shown in figure 2B. Thus, the strip may comprise a sample receiving pad (1), said pad comprising an element for conjugate storage (2), followed by a membrane (3) comprising a detection area (4). In one embodiment, this combined pad (1,2) is located before the membrane (3) and is in communication with it, more preferably, the combined pad (1,2) is overlying partially or totally the membrane (3) or is overlaid partially or totally by the membrane (3). In a further embodiment the combined pad (1,2) is mounted on the membrane (3).

According to another configuration, sample and conjugate are pre-mixed. Sample and conjugate may be pre-mixed before applying the sample to the device of the invention (i.e. using a liquid conjugate indicator; no conjugate area is therefore needed). In one embodiment, as shown in figure 2C, the sample application area (1) is located before the membrane (3) and is in communication with it, more preferably, the sample pad (1) is overlying partially or totally the membrane (3) or is overlaid partially or totally by the membrane (3). In a further embodiment the sample pad (1) is mounted on the membrane (3). In an alternative embodiment, the membrane (3) comprises a sample application area (1) on which a sample pre-mixed with conjugate is applied and a detection area (4), as shown in figure 2D.

Furthermore, it is contemplated that the sample application area (1) is adapted to the sample which is intended to be analyzed. The sample pad is usually impregnated with buffer salts, proteins, surfactants and other liquids to control the flow rate of the sample and to make it suitable for the interaction with the detection system. According to one embodiment, the sample application area (1) is impregnated with bovine serum albumin (BSA) and surfactants.

Moreover, the pores of the sample pad can act as a filter in order to remove redundant materials. For assaying blood samples, for example, the sample pad may additionally include materials that can separate blood cells. Filtered sample can then reach the conjugate area, if present, or directly move towards the detection area. The blood separation pad can also function as an adsorbent that removes interfering factors from the sample by adsorption. There are several blood separation pads commercially available, for example, MDI® FR-1 (0.6) pad, MDI® FR-1 (0.35) pad, Whatman® MF-1 pad, Ahlstrom® Cytosep 1660 pad, Ahlstrom® Cytosep 1662 pad, paper pad treated with PHA, or the like. Nevertheless, thanks to the electrophoretic separation, in the device of the invention the need of filters is advantageously avoided or minimized.

The term "conjugate" as used herein means any moiety bearing both a label or reporter (indicator element) and a binding partner (recognition element), that can be used to determine the presence of or an amount of an analyte. The label is a material detectable at the detection area (4). The binding partner is a material that can bind or compete with the analyte. Example binding partners included in conjugates include antibodies, antigens, analyte or analyte-mimics, protein receptors, enzymes, aptamers, oligonucleotide probes, etc. The conjugate can take two distinct forms, depending on whether the assay is designed to exploit the "sandwich" or "competitive" technique.

In the case of the sandwich assay, the binding partner in the conjugate can form a complex including the analyte and the conjugate and that complex can further bind to another binding partner, also called a capture element, integrated into the detection zone (4), to form a "sandwich". This sandwich complex is progressively produced at the test site (4a) as sample continuously passes by, producing a detectable signal. For instance, in certain embodiments, the complex may be detected by visual observation of color development at the test site (4a). As more and more conjugate is immobilized, the colored particles accumulate at the test site (4a) and become visible through the window, indicating the presence of ligand in the liquid sample.

In the case of the competitive technique, the analyte will interfere with binding of the binding partner in the conjugate to another binding partner, also called a capture element, integrated into the detection zone (4). That is the conjugate binds with the capture element in competition with the ligand (analyte). The binding partner in the conjugate may comprise, for example, an authentic sample of the ligand or a fraction thereof which has comparable affinity for the capture element. As the liquid sample is transported in contact with the test site (4a), ligand, if any, and the conjugate compete for sites of attachment to the capture element. If no ligand is present, colored particles (or other kind of detectable particles) aggregate at the test site (4a), and the presence of color (or any other signal) indicates the absence of detectable levels of ligand in the sample. If ligand is present, the amount of conjugate which binds at the test site (4a) is reduced, and no color, or a paler color, (or no signal, in general) develops.

As noted above, in one embodiment of the invention, the test sample may be mixed with the conjugate outside the device. In another embodiment, the conjugate is disposed in freeze-dried or other preserved form on the permeable material between the inlet of the device and the test site (4a), and the sample liquid resolubilizes the conjugate as it passes along the flow path.

The conjugate area (2), if present, is normally formed by or comprises a conjugate pad that comprises the labelled recognition element that is released by the liquid flow to interact or compete with the analyte and to bind to the test or control line in the detection area. Once the sample is applied onto the application pad (1) and an electric potential is applied, the sample flows in to the conjugate release pad (2) where it releases the conjugate; the conjugate then leaves the conjugate release pad and moves with the sample in to the membrane (3).

Conjugate pads employed in a conventional lateral flow assay device may be used in the present invention. It is thus contemplated that the conjugate pad (2) is made of a bibulous, porous or fibrous material capable of absorbing liquid rapidly. The porosity of the material can be unidirectional (for example, with pores or fibres running wholly or predominantly parallel to an axis of the structure) or multidirectional (for example, omnidirectional, so that the, member has an amorphous sponge-like structure). Porous structure can be made for instance from non-woven glass fiber, paper or other cellulosic materials, such as nitrocellulose. Porous plastics material, such as polypropylene, polyethylene, polyvinyliderie flouride, polyester, ethylene vinylacetate, acrylonitrile and polytetrafluoroethylene can also be used. It can be advantageous to pre-treat the material with a surface-active agent during manufacture, as this can reduce any inherent hydrophobicity in the material and, therefore, enhance, its ability to take up and deliver a moist sample rapidly and efficiently. In a preferred embodiment, the conjugate pad (2) is made of glass fiber or polyester.

In an embodiment, said conjugate pad (2) is positioned in communication with the membrane (3) and, more preferably, said conjugate pad (2) is mounted on the membrane overlying partially or totally the membrane (3).

It is contemplated that the conjugate is deposited in the conjugate area or pad (2). In one embodiment, said conjugate is deposited in the pad superficially forming, thus, a superficial layer. In another embodiment, the conjugate applied in the pad (2), can penetrate until the membrane (3) and, in some, cases it may also penetrate said membrane (3). In an alternative embodiment, the conjugate is directly applied in the membrane (3).

The conjugate may be placed in the membrane (3) or in the conjugate pad (2) by means of techniques and procedures known by the person skilled in the art. In the most preferred embodiment, said conjugate is sprayed, spotted or applied by a mechanical liquid dispenser or by immersion over the conjugate area (2) positioned in the membrane (3) or in the conjugate pad (2), more preferably sprayed. Then, the conjugate applied may be dried. Said drying may be performed for instance at about 37 °C during at least about 20 minutes.

A pre-treatment may be necessary to increase wettability, decrease non-specific interactions and to control the pH.

According to one embodiment, the conjugate area or pad (2) is treated with impregnation solution typically containing polymers, surfactants, and proteins to achieve effective conjugate release properties and rapid wettability.

According to one embodiment, a conjugate buffer, containing carbohydrates (such as sucrose), is used as a preservative and a resolubilization agent. When the conjugate particles are dried in the presence of sugar, the sugar molecules form a layer around them stabilizing their biological structures. When the sample enters the conjugate pad, the sugar molecules rapidly dissolve carrying the particles into the fluid stream.

The recognition element employed in the assay typically consists of antibodies or antigents which have been conjugated or labeled with any moiety or reagent that allows detection in the detection (area), preferably direct detection, i.e. without needing to apply further reagents, reducing, thus, manipulation of the sample and the number of interactions or reactions required to obtain a detectable signal. Said detectable label can be covalently or non-covalently bound/coupled to the recognition element. The binding/coupling can be accomplished by any method known in the art.

Examples of recognition elements include, but are not limited to, aptamers, oligonucleotide probes, enzymes, protein receptors, monoclonal or polyclonal antibodies, and recombinant or native antigens. Most commonly, antibodies are used as the analyte-binding molecules. Antibodies may be designed and highly purified so as to obtain a consistent antibody supply with proven specificity and affinity. However, antigens may be used as the binding reagents in assays detecting antibodies against specific pathogenic molecules.

Suitable detectable labels include both optical and non-optical reporters. Examples of detectable labels, but are not limited to, colored, fluorescent, electrochemical and magnetic particles as well as enzymes.

In a preferred embodiment, a colored label is used such as a metal colloidal particle, more preferably a gold colloidal particle, for example, gold microparticles or gold nanoparticles. Gold particles, also known as colloidal gold, are the most commonly used reporter technology. Although colloidal gold can be prepared in the laboratory at low cost, there are many commercial sources available. It has an intense colour and no development process is needed for visualization. Moreover, it has high stability in both liquid and dried forms. Gold colloidal particles used as a detectable label may have for instance a diameter of between 20 nm and 80 nm, even more preferably, of between 20 m and 40 m.

Gold particles produce a colored readout which requires no development process for visualization. Further, the inherent physical characteristics of gold also enables the measuring of the colloidal gold in LFAs with surface-enhanced Raman spectroscopy (SERS) or localized surface plasmon resonance (LSPR).

The typical analytical sensitivities achieved using visual detection of reporters in LFAs ranges from to 3 pM to 10 µM. Various methods for improving the assay sensitivity with visually detectable reporters have been described. The detectability of colloidal gold can be improved up to 400-fold by using an electrokinetic pre-concentration method or up to 500-fold by using a silver enhancement method to increase the light absorptivity of colloidal gold.

Another label that may be used is latex. Latex can be tagged with a variety of detector reagents such as coloured or fluorescent dyes, and magnetic or paramagnetic components. As latex can be produced in multiple colours, it has an application in multiplex assays, which require discrimination between numerous lines.

Visually detectable reporters also include the enzymatic reactions used to generate a visible substance at the test line (4a) where the immunocomplex formation occurs. The presence of analyte can be indicated by tracer antibodies having the colour-reaction catalysing enzymes conjugated with antibodies or alternatively enzymes produced as fusion proteins. Such technology has been described most commonly using horseradish peroxidase (HRP) as the catalyst enzyme. Enzyme-coupled tracer antibodies are not only used as visually detectable reporters, but also used in electrochemical detection in LFAs.

Carbon nanoparticles have been reported as a reporter technology well suitable for rapid diagnostic assays. The main benefits of the technology over the colloidal gold are better contrast on the white nitrocellulose membrane and low-cost manufacturing process. The carbon nanoparticles have been reported to yield better analytical sensitivities compared to colloidal gold or latex. For instance, carbon nanotubes have been shown to exhibit a limit of detection that is 10-fold lower than that of gold. Thus, sensitivities comparable to fluorescent reporters can be achieved using carbon nanoparticles. In addition to carbon nanoparticles used as visually detectable reporters there are also fluorescent carbon nanoparticles that have been used for cell imaging.

Fluorescent reporters such as Europium(III) nanoparticles or quantum dots may also be used. It is possible to exploit the quenching capability of fluorescent reporters to generate analyte-specific assay response. For instance, quantum dot reporters may be conjugated with analyte-specific antibodies and immobilized to the test line (4a) of the strip and graphene oxide may be used to reveal the antigen binding to the quantum dot particle, as graphene oxide is only able to quench the fluorescence from quantum dots not masked by the bound antigen. Such a method may be advantageous for multiplex assays, as there would be no need to dry multiple different reporter bioconjugates on the conjugate pad (2). Examples or quantum dots include, but are not limited to, CdSe@ZnS QDs (optionally functionalized with streptavidin), CdTe QDs and G-QDs (graphene QDs).

The amount of reporter particles retained on the test line (4a) can be measured also with a magnetic field. The magnetic field induces a measurable modulation in the radio frequency (RF) signal in close proximity to the test line (4a). The magnetic detection may shown potential for the production of high detection sensitivity.

Different electrochemical detection technologies have also been reported with LFAs and could be used in the present invention. The change of electric conductivity or the generated current in the presence of certain reporters can be measured with simple electrodes attached to the membrane.

Detection of the detectable label will depend on the chosen moieity or reagent, and can be done by any of the methods known in the state of the art, for example, visual inspection, ultraviolet and visible spectrophotometry, fluorimetry, radioactivity counting or the like. In a preferred embodiment, for instance when gold particles are used as detectable labels, the results of the assay may be obtained and analyzed by visual inspection.

In a particular embodiment, a colorimetric label and/or a fluorescent label are used. In a more particular embodiment, gold nanoparticles (AuNP) and quantum dots (QDs) are used as labels (indicator element).

The device of the invention may contain one or more light sources, the light sources being capable of transmitting at least one wavelength of light configured to yield a detectable signal from the reporter(s). In a particular embodiment, the device of the invention contains one or more LEDs to provide excitation for a fluorescence emission of a fluorescent label and one or more white LEDs for improving a colorimetric detection. In a more particular embodiment, the device of the invention contains two LEDs to provide excitation for a fluorescence emission of a fluorescent label and two white LEDs for improving a colorimetric detection.

Passive adsorption methods can used to attach antibodies to detection moieties. The inherent protein-binding feature of the colloidal offers a convenient way to produce a reporter bioconjugate. The optimal amount of protein used in the conjugation can be determined by the amount of protein that prevents the visible aggregation of gold particles. However passive adsorption can be time-consuming and requires specialist knowledge. Covalent conjugation allows the production of highly stable conjugates, and uses considerably less antibody than passive adsorption methods. For reporter particles with carboxyl-modified surface a covalent linking of the biomolecule and the reporter may be done for instance by carbodiimide linkage using N-(3-Dimethylaminopropyl)-N'-ethylcarbodiimide hydrochloride (EDC) and N-hydroxysuccinimide (NHS) as a stabilizing agent. Covalent protein conjugation with activated latex is a common method that also enables controlled orientation of the antibody in the conjugation. The direct conjugation of antibodies or proteins to gold nanoparticles or latex microspheres can be carried out quickly and easily using InnovaCoat® GOLD and LATEX conjugation kits from Innova Biosciences.

Fluid electrophoretically flows from sample application area (1), optionally via the conjugate area (2), downstream to the capture detection area (4) of the membrane (3). Membranes employed in a conventional lateral flow assay device may be used in the present invention. Therefore, as conventional LFA devices, the LEB device of the present invention is a paper-based sensor, wherein the terms "paper" or "paper-based" is intended to mean that the membrane comprises cellulose or a derivative thereof such as nitrocellulose. Suitable materials for the membrane may be selected for instance from the group consisting of nitrocellulose, cellulose, cellulose acetate, cellulosic paper, cellulose nanopaper (CNP), chromatography paper, filter paper, tissue paper, writing paper, and paper towel. Other materials such as polyvinylidene fluoride (PVDF), nylon, and polyethersulfone (PES) may also be employed as porous membrane. In a preferred embodiment, the porous membrane is a nitrocellulose membrane. For instance, nitrocellulose membranes with a size of about 6.8 cm x about 0.8 cm can be used as platform for running the electrophoresis. Nitrocellulose membranes exhibiting a range of pore sizes (0.05 to 12µm) are commercially available, but since the pores do not have an even distribution it is more appropriate to consider capillary flow time when selecting a membrane for the lateral flow immunoassay. Capillary flow time is typically expressed as seconds/cm and is defined as the time taken for the sample liquid to travel to and completely fill the strip of membrane. In a particular embodiment, the membrane is a nitrocellulose membrane with a capillarity flow rate ranging from about 240s/cm² to about 075 s/cm². In a more particular embodiment, the membrane is a nitrocellulose membrane HF240, HF180 and HF075 (with a capillarity flow rate of 240s/cm², 180s/cm² or 075s/cm², respectively).

The membrane strip may be patterned to delimitate the flow area, for instance by wax printing.

The term "capture element" is intended to mean any chemical, biochemical or biological entity capable of binding specifically with the analyte.

Capture elements (e.g. antibodies) are immobilized across the membrane (3) in the detection area (4) at least in one line (test line, 4a) and typically in two lines (test and control line, 4a and 4b). Test line (4a) comprises at least one capture agent immobilized on membrane that is specific for the analyte of interest.

In the "sandwich" approach, capture elements at the test line (4a) are binding partners capable of binding to the complex analyte-labelled recognition element to form a "sandwich". The presence or absence of the analyte in the liquid sample are measured, respectively, by the presence or by the absence of a visible signal (or measurable) at the test line (4a).

In the "competition" approach, capture elements at the test line (4a) are binding partners capable of binding in competition with either the analyte of the sample or with the labelled recognition element. The presence or absence of the desired analyte in the sample are measured, respectively, by the absence or the presence of a visible signal (or measurable) at the test line (4a).

Capture agents that may be effectively employed in the disclosed device are well known to those of skill in the art and include antigens, anti-bodies, nucleic acid molecules, and other relevant protein or non-protein molecules. For example, the capture agent may comprise an antibody that specifically binds to a known disease antigen. Multiple test lines (4a) may be employed depending on the number of analyte-specific detection agents to be incorporated, and/or to enable testing for more than one analyte. In particular embodiments, one, two, three or more test lines are employed.

Control line (4b) is also normally present and is used as an internal control to ensure that all the test components are working. Control line (4b) comprises entities that bind to the recognition agent irrespective of the presence or absence of the analyte. For example, for antigen antibody interactions, control line (4b) may comprise anti Protein A or human IgG immobilized on membrane.

Thus, test lines (4a) and control lines (4b) contain capture reagents to display the result. No binding at the test lines (4a) depicts a negative result. Visual control line (4b) indicates test has run correctly.

This device of the invention may utilize paper-based test strips comprising a pair of electrodes of opposite charge (cathode and anode) located at or close to the end parts of the strip so that the sample application area (1), optionally the conjugate area (2) and the detection area (4) are disposed between the electrodes. These paper strips may comprise further a pair of wicks or pads to storage electrolytic solution in fluid communication with the electrodes. Figure 16 shows an embodiment of a paper strip comprising two electrodes (7) and two cellulose pads (8) at its ends. All the necessary reagents (e.g. conjugate and capture elements) and even the electrodes and the wicks for electrolytic storage may be included within disposable paper-based strips.

The strip test device of the present invention may be configured for receiving and fixing one or more paper strips. The use of more than one paper strips is advisable for instance for a more rapid calibration for quantitative analysis. Preferably, said one or more paper strips are substantially parallel disposed. In particular embodiments, one, two, three or more paper strips are employed.

The device further comprises means for producing an electrical potential difference across the paper strips. According to one embodiment, said means comprise:
- an electrical power source or a connector adapted to connect the device to an external electrical power source;
- at least a pair of electrodes of opposite charge connected to the power source or to the external power source connector, and being disposed spaced apart from each other so that one or more electrodes are in fluid communication with one or more paper strips in a region located before the application sample area thereof and the opposite electrode or electrodes are in fluid communication with the one or more paper strips in a region located after the detection area thereof; and
- means for providing electrolytic solution so as to keep the electrodes and the paper strips in contact with electrolytic solution;
so that in operative manner an electric field is generated between the opposite electrodes across the paper strips.

Thus, the electrophoretic system comprises: electrical power supply, at least a pair of spaced electrodes, one or more paper strips as support medium and electrolytic solution contacting the electrodes and the paper strips. More particularly, the electrophoretic system comprises at least a pair of spaced electrodes, one of said spaced electrodes acting as a cathode and the other as an anode, wherein said electrodes are connected to the power source or to the external power source connector and are arranged at or close to opposite end parts of the paper strips so that the sample application area (1), optionally the conjugate area (2) and the detection area (4) are disposed between the electrodes, wherein both the electrodes and the paper strips are immersed, impregnated or otherwise in contact with electrolyte.

In this way, the electrophoretic system makes the sample migrate from the application area (1) to the detection area (4) when a potential is applied. More particularly, the sample is deposited upon sample area (1) of one or more strips and are subjected to an electric field by maintaining an electrical potential difference between the pair or pairs of electrodes, to have the components of the sample undergo an electrophoretic migration, optionally via a conjugate area (2), towards the detection area (4). The analyte of the sample can be subsequently rendered detectable in the detection area (4) by visual inspection or by other methods.
According to one embodiment, there is one pair of electrodes of opposite charge for one or more paper strips, and said pair of electrodes of opposite charge is in fluid communication with the one or more paper strips. According to another embodiment, there is a pair of electrodes of opposite charge for each paper strip and each pair of electrodes of opposite charge is in fluid communication with one paper strip.

Suitable electrode materials include, independently, non-noble metals, such as one or more of the alkali metals, alkali earth metals, transition metals, noble metals, lanthanides, actinides, post-transition metals, and combinations (alloys) thereof. Exemplary metals include, copper, gold, silver, iron, tungsten, aluminum, titanium, platinum, palladium, vanadium, and nickel), metal alloys of iron (for example, low carbon steels, cast iron), alloys of titanium, alloys of copper/tin.

The electrodes can be configured as bilayer or multilayer electrodes. For example, bilayer electrodes can be made up of an outer layer of gold and an inner core of copper (or visa-versa), as a heterogeneous distribution, and/or having a substantially outer layer of a first metal and partially surrounded, yet partially exposed sections of another metal. Multi-layer electrodes can be made up for instance of alternative layers or longitudinally arranged sections of two or more metals suitable for preparing multi-metal compositions. In a particular embodiment, copper electrodes covered with a gold layer are used.

The electrodes may be configured with fixing elements, e.g. as a retaining clip, for securing the paper strips to the device.

Examples of electrolytic (buffer) solution include, but are not limited to acetic acid, boric acid, phosphoric acid and citric acid as well as glycine and taurine. Generally, the pKa value (acid dissociation constant) should be close to the required pH. It is preferable to use buffers that provide a low charge magnitude so as not to conduct too much current.

The electrolytic solution may be stored for instance in reservoirs, in wicks or the like, adapted to provide electrolytic solution to the electrodes and to the paper strips. In a particular embodiment, the means for providing an electrolytic solution to the electrodes and to the paper strips comprise:
- at least a pair of reservoirs to storage electrolytic solution, each reservoir of the at least a pair of reservoirs being adapted to maintain each electrode of the at least a pair of electrodes and each end part of each paper strip submerged in the electrolytic solution; or
- at least a pair of wicks to storage electrolytic solution, each wick of the at least a pair of wicks being adapted to maintain each electrode of the at least a pair of electrodes and each end part of each paper strip impregnated with the electrolytic solution.

According to one embodiment, the electrolytic or buffer solution is stored in storage wells or reservoirs containing the electrodes and each end part of the strip is maintained in contact with the electrolytic solution by direct immersion in the solution contained in the reservoirs. The buffer then flows by capillarity along the paper strip acting as a bridge between the electrodes. The device of example 1 represents a possible configuration according to this embodiment. In particular, the device of example 1 includes three LFA strips and each end part of them is immersed in a well containing one electrode and buffer solution. The well plate made be for instance of Teflon and nylon.

According to another embodiment, the electrolytic or buffer solution is stored in storage pads or wicks (e.g. cellulose pads) contacting the electrodes which in turn contact each end part of the strip. The buffer then flows by capillarity along the paper strip acting as a bridge between the electrodes. The device of example 2 represents a possible configuration according to this embodiment. In particular, the device of example 2 uses a paper strip comprising two electrodes (7) and two cellulose pads (8) at its ends.

Thus electrodes are immersed, impregnated or otherwise in contact with electrolytic solution and in fluid communication with the opposite end parts the paper strips so that electrolytic solution can flow to the paper strips to allow a desired voltage gradient to be maintained across the strips when direct current power is applied to the electrodes.

According to one embodiment, there is one pair of electrodes of opposite charge and one pair of means for providing electrolytic solution (e.g. reservoirs, wicks or the like) for one or more paper strips, said pair of electrodes of opposite charge is in fluid communication with the one or more paper strips and each electrode is immersed, impregnated or otherwise in contact with one means for providing electrolytic solution.

According to another embodiment, there is a pair of electrodes of opposite charge and a pair of means for providing electrolytic solution (e.g. reservoirs, wicks or the like) for each paper strip, each pair of electrodes of opposite charge is in fluid communication with one paper strip and each electrode of each pair of electrodes is immersed, impregnated or otherwise in contact with one means for providing electrolytic solution. In a preferred embodiment, the device of the invention is powered by an external electrical power source. In a particular embodiment, the device of the invention comprises a connector adapted to connect to an external electrical power source, said connector comprising an adapter to increase the voltage of the external electrical power source.

One of the main limitations of the conventional electrophoresis systems is the need of a high voltage supply requiring the use of a bulky power source; to overcome this problem the device of the invention may be powered by a portable computing device, such as a smartphone, connected to a Joule thief inverter and a rectifier (see Figure 7). Although it could be expected that the low intensity of the output current is not enough to perform an electrophoresis, advantageously the high resistance of the system (several mega ohms) allows to keep a constant voltage in the device (240v DC). In this way, the end user can perform a quick assay at anytime, anywhere only by using a smartphone orany other similar device.

Thus, in a preferred embodiment, a portable computing device (e.g. a smartphone) is used as a power source for the device of the invention. More preferably, the device of the invention is coupled to a portable computing device by means of Joule thief inverter and a rectifier (see Figure 7). The Joule thief inverter acts as a voltage booster and changes incoming direct current (DC) to alternating current (AC). The rectifier converts back alternating current (AC) to direct current (DC). In this way, it is possible to convert an output 5v DC voltage from a portable computing device into a 240v DC voltage.

In a more preferred embodiment, a portable computing device (e.g. a smartphone) is used both as optical readout and as a power source for the device of the invention.

The term 'portable computing device' as used herein encompasses any device operating on a limited capacity power supply, such as a battery, and that can be moved from one location to another. More particularly, the term 'portable computing device' refers to any handheld battery powered mobile appliance, including but not limited to a cellular telephone, a satellite telephone, a pager, a personal digital assistant ("PDA"), a smartphone, a navigation device, a smartbook or reader, a tablet computer or a laptop computer or a combination of the aforementioned devices. The 'portable computing device' preferably comprises a camera for the detection readout. The pictures taken by the camera may be evaluated for instance using an appropriate software such as Image J software. The device of the invention may be connected for instance by an OTG wire to a smartphone or other portable computing device.

The container can be made of any material know in the art that allows the protection of the strip. For instance, the container may be made of plastic such as polylactic acid. The container may be made using a 3D printer.

In addition, it is noted that the container has a structure that may allow the application of the sample and the visualization of the results of the test without removing said container. For instance, referring to the embodiment of figure 16, the cassette for housing the strip comprises one orifice for applying the sample onto the sample application area (1), a reading window for visualizing the test and control lines (4a, 4b), two orifices for applying the buffer onto the cellulose pads (8) and two orifices for the electrode connections (9).

In an additional embodiment, the device of the present invention further comprises a lancet and a blood collection component.

The invention also relates to the use of the device of the invention for the detection, and optionally quantification, of an analyte in a sample. In a particular embodiment, said use relates to a point-of-care medical testing of a disorder or condition such as infectious diseases, pregnancy, microbial infections, cancer, autoimmune disorders, cardiac disorders, allergic disorders, drug abuse, and the like.

The invention also relates to an in vitro method of prognosis, diagnosis, treatment, post-treatment monitoring and/or decision-making in a subject, preferably a mammal including a human, suspected of having, having or susceptible to having a disorder or condition such as infectious diseases, pregnancy, microbial infections, cancer, autoimmune disorders, cardiac disorders, allergic disorders, drug abuse, and the like, which comprise detecting the presence of at least one marker in a biological sample of the subject with a device according to the present invention. More particularly, said method may comprise:
- dispensing a biological sample from the subject onto the application area (1) of the one or more paper strips, wherein said sample either has been previously treated with conjugate outside the device or is treated with conjugate present on the device, said conjugate comprising at least a labelled recognition element specific for or competitive with the at least one marker;
- producing an electrical potential difference across the one or more paper strips so that the sample starts migrating along the one or more paper strips by electrophoresis; and
- continuing the migration of the sample towards a detection area (4) of the one or more paper strips so that the presence of marker may be determined by a detectable signal at the detection area (4), wherein the detection or the non-detection of said detectable signal is indicative of the disorder or condition.

In a particular embodiment, the method is also suitable for the quantification of the at least one marker in the biological sample of the subject (test sample), said method further comprising obtaining a calibration curve from calibration samples containing known or allocated amounts of marker and quantifying the marker in the test sample using the calibration curve and the value of the signal generated with the test sample. The invention can include quantifying the target analyte in the test sample algebraically using signals generated by one or more calibration samples.

The amount of marker in the test sample is indicative of the status of the disorder or condition and may be compared with a reference value. "Reference value", as used herein, refers to a laboratory value used as a reference for values/data obtained by examinations of samples tested according to the present invention. The reference value or reference level can be an absolute value; a relative value; a value that has an upper and/or lower limit; a range of values; an average value; a median value, a mean value, or a value as compared to a particular control or baseline value. A reference value can be based on an individual sample value, such as for example, a value obtained from a sample from the subject being tested, but at an earlier point in time or from a healthy tissue. The reference value can be based on a large number of samples, such as from population of subjects of the chronological age matched group, or based on a pool of samples including or excluding the sample to be tested. Various considerations are taken into account when determining the reference value of the marker. Among such considerations are the age, weight, sex, general physical condition of the patient and the like. For example, equal amounts of a group of at least 2, at least 10, at least 100 to preferably more than 1000 subjects, preferably classified according to the foregoing considerations, for example according to various age categories, are taken as the reference group.

Once this reference value is established, the level or quantity of marker can be compared with this reference value, and thus the status of the condition or disorder may be determined. For instance, in certain instances, a level of marker above (e.g. at least 1.1-fold, 1.5-fold, 5-fold, 10-fold, 20-fold, 30-fold, 40-fold, 50-fold, 60-fold, 70-fold, 80-fold, 90-fold, 100-fold or even more) a reference value may be indicative of low risk of developing a condition or disorder (prognosis), of not having a condition or disorder (diagnosis), of a good response to therapy of a patient having a condition or disorder (post-treatment monitoring), etc. In other instances, a level of marker below (e.g. at least 0.9-fold, 0.75-fold, 0.2-fold, 0.1-fold, 0.05-fold, 0.025-fold, 0.02-fold, 0.01-fold, 0.005-fold or even less) a reference value may be indicative of low risk of developing a condition or disorder (prognosis), of not having a condition or disorder (diagnosis), of a good response to therapy of a patient having a condition or disorder (post-treatment monitoring), etc.

Based on the test results, the invention also relates to a method of treatment of a subject, notably a human, having or susceptible to having a disorder or condition such as the abovementioned ones, which comprises administering to the subject in need of such a treatment or prophylaxis an effective amount of a drug useful for treating or preventing said disorder or condition.

By an "effective" amount of a drug is meant a nontoxic but sufficient amount of the drug to provide the desired effect. The amount that is "effective" will vary from subject to subject, depending on the age and general condition of the individual, the particular active agent or agents, and the like. However, an appropriate "effective" amount in any individual case may be determined by one of ordinary skill in the art using routine experimentation.

Alternatively, the developed electrophoresis device of the invention may be applied for the characterization of biomolecules, nanomaterials and biomolecule-nanomaterial conjugates, in order to determine their surface charge and size. This is currently done by gel electrophoresis, in which the biomolecules/nanomaterials move to the positive or negative electrode at a specific rate according to their surface charge and size. For example, molecules have an individual isoelectric point that is the pH at which they do not have a net electrical charge. This charge is affected by the molecule's surrounding pH, in the sense that an increase or decrease of the pH will cause the molecule a gain or loss of protons. The same phenomena is observed in nanomaterials, in which their surface charge is related to the functional groups present in the surface and will vary depending on the pH of solution. In this context, electrophoresis is widely applied for the evaluation of the conjugation of nanomaterials to biomolecules. Briefly, nanomaterials are used as signal transducers in biosensing applications. The nanomaterials are linked to biorecognition elements, such as antibodies or DNA probes and this is known as bio-conjugation. A deep characterization of the bio-conjugation procedure is required before performing the bioassay, and this is normally done by evaluating an absorbance shift with spectrophotometer or a change in size and charge with DLS or electrophoresis. The latter, is due to the change in the surface charge of the nanomaterial due to the addition of biorecognition element, which has its own surface charge. Therefore, one way to determine if the bioconjugation has been achieved is performing an electrophoresis of the bioconjugated nanomaterial, in which a difference in its electrophoretic mobility is expected.

The developed electrophoresis device, although based on paper substrate, has the capability of separating nanomaterials by its differences in surface charge and size and this is proved in figure 4 and 5. Thus, the device of the invention can be used as a fast and simple method for determining the surface charge of a biomolecule/nanomaterial or checking if its functionalization has been effective.

In another aspect, the present invention refers to a kit to carry out the method of the present invention comprising:
- a device as described above;
- instructions to carry out the assay; and
- optionally one or more paper strips.

The skilled person knows that numerical values relating to measurements are subject to measurement errors which place limits on their accuracy. Where terms such as "about" or "approximately" are applied to a particular value (e.g. "about 200 °C" or "approximately 200 °C") or to a range (e.g. "about x to approximately y"), the value or range is interpreted as being as accurate as the method used to measure it. Unless explicitly stated otherwise, the general convention in the scientific and technical literature may be applied so that the last digit of numerical values preferably indicates the precision of measurement. Thus, unless other error margins are given, the maximum margin is preferably ascertained by applying the rounding-off convention to the last decimal place. For instance, a value of 3.5 preferably has an error margin of 3.45 to 3.54 and a range of 2% to 10% preferably covers a range of 1.5% to 10.4%. Said variations of a specified value are understood by the skilled person and are within the context of the present invention. Further, to provide a more concise description, some of the quantitative expressions given herein are not qualified with the term "about". It is understood that, whether the term "about" is used explicitly or not, every quantity given herein is meant to refer to the actual given value, and it is also meant to refer to the approximation to such given value that would reasonably be inferred based on the ordinary skill in the art, including equivalents and approximations due to the experimental and/or measurement conditions for such given value.

It should be understood that the scope of the present disclosure includes all the possible combinations of embodiments disclosed herein.

The following examples are merely illustrative of certain embodiments of the invention and cannot be considered as restricting it in any way.

### EXAMPLES

Studies were performed to assess that the LEB system of the invention works and to compare its effectiveness with that of a conventional lateral flow assay device.

### EXAMPLE 1

A LEB device was designed to fulfil all requirements according to PoC tests. The developed device was powered by a smartphone connected to a Joule thief inverter and a rectifier (Figure 7).

The LEB device was made of polylactic acid using a 3D printer. Since polytetrafluoroethylene (Teflon) is more resistant to leakage, this material was used to store the buffer solution. Copper electrodes covered with a gold layer were coupled to the platform (Figure 8). Considering the advantages of the nitrocellulose as a cost-effective, flexible and renewable material with a well controlled porosity, a nitrocellulose membrane with a size of 6.8 cm x 0.8 cm was used as platform for running the electrophoresis. The end of the strip was immersed into the buffer solution containing the electrolytes and providing the ionic strength that supports the mobility of the charged particles along the paper strip enabling an electric bridge between the cathode and anode. Furthermore, the nitrocellulose membrane strip was patterned with a wax printer delimiting the flow area.

Since optical detection methods were used, the LEB device was designed to be in a light controllable environment requiring a cover to remove possible background signals. The cover was 3D-printed and contains four LEDs, two to provide an excitation wavelength of 365 nm, for the fluorescence emission (655 nm) of the CdSe@ZnS QDs and two white LEDs for the colorimetric approach using AuNP (Figure 9). The smartphone also powered the LEDs and its camera enabled the detection readout.

Figure 3 illustrates the working principle of the LEB according to this example. A serum sample that contains a target analyte and other interfering proteins is incubated with antibody-conjugated nanoparticles (quantum dots, QDs, or gold nanoparticles, AuNP). The mixed suspension is drop-casted onto a nitrocellulose membrane (Figure 3A). After applying a potential, the proteins, the labelled antibodies and the remaining serum compounds are forced to move due to electrophoresis. The difference in surface charge of the sample components enables its separation and therefore the purification of the target analyte from the interfering proteins. Moreover, the target analyte is recognized by the antibody-conjugated QDs, which force the movement of the analyte towards the detection zone (Figure 3B). The schematic representation of the developed device is shown in Figure 3C.

### Methods

### LEB device design and fabrication

The LEB device was designed using Tinkercad software and fabricated in separate pieces. The 6 well plate was made of Teflon and nylon using a three-axis machined centre. Each well has a dimension of (1.2 x 1.2 x 1.2 cm) and contained a copper electrode (covered with gold) (see Figure 9 for the connection diagram). The nitrocellulose membrane support and the device cover and base were made of polylactic acid and fabricated using a 3D printer (BCN3D Sigma). Two LEDs (365 nm excitation wavelength) and two white LEDs were introduced inside the device cover and connected by an OTG wire to a Smartphone (Samsung Galaxy S7 Edge). The electrophoresis device had a final dimension of (14 x 8 x 10 cm).

### Nitrocellulose membrane strip fabrication

The nitrocellulose membranes used for the evaluation of the electrophoresis performance were HF240, HF180 and HF075 (Millipore). For the detection of H-IgG, CN95 nitrocellulose membrane was used. Polyclonal goat to H-IgG antibodies were deposited in TL and polyclonal rabbit to sheep IgG antibodies in CL, using a biospoter (Biofluidix Biospot Workstation). The antibodies were dried for 2 hours at 37 °C and a channel of (7.7 x 0.3 cm) was patterned using a wax printer (ColorQube 8570).

### Evaluation of the electrophoresis performance

The streptavidin functionalized CdSe@ZnS QDs (Q10121MP) were purchased in Thermofisher. The CdTe QDs were purchased in Plasma Chem (Hydrophilic CdTe Quantum Dots Kit). The G-QDs were synthesized homemade following the procedure of Dong, et.al. [Dong, Y. et al. Blue luminescent graphene quantum dots and graphene oxide prepared by tuning the carbonization degree of citric acid. Carbon 50 (12), 4738-4743 (2012)]. The N-S-doped G-QDs were synthesized following the procedure reported by Dong, et.al. [Dong, Y. et al. Carbon-based dots co-doped with nitrogen and sulfur for high quantum yield and excitation-independent emission. Angew Chem Int Ed Engl 52(30), 7800-7804 (2013)]. The evaluation of the effect of the buffer pH on the electrophoretic mobility of the QDs was carried out introducing into each well 1 mL of sodium borate buffer (10mM, pH 8) and adjusting the pH with boric acid (B-7901-500G, Sigma-Aldrich) and sodium hydroxide (131687, PanReac).

### Detection of H-IgG in PBS and human serum

The AuNps were synthesized by the Turkevich method [Turkevich J. *et al.* A study of the nucleation and growth processes in the synthesis of colloidal gold. *Discuss Faraday Soc* **11**, 55-75 (1951)] and characterized by TEM (see figure 15) and UV-Vis spectrum. They were conjugated with antiH-IgG following the experimental procedure already reported by the group [Ambrosi, A. et al. Double-codified gold nanolabels for enhanced immunoanalysis. Anal Chem 79, 5232-5240 (2007)]. The streptavidin functionalized CdSe@ZnS QDs (1 µM) were diluted to 25 nM using immunobuffer (10 mM PBS pH 7.4, 1% BSA, 0.5 % Tween 20). The polyclonal sheep anti-HIgG (ab6869, Abcam) was diluted to 2 µg/mL using immunobuffer. They were both mixed in a 1:1 volume ratio and incubated at 350 rpm and room temperature for 1 hour. The H-IgG (12511-10MG, Sigma-Aldrich) was spiked in human serum (CLP-HS2001-25-25mL, Vitro S.A.). 3 µL of the bioconjugated CdSe@ZnS QDs were mixed with 3 µL of the H-IgG-serum solution, incubated for 30 seconds and drop-casted in the centre of the nitrocellulose strip. The battery is switched on and the assay is carried out until the TL and CL are clearly visualized.

### Evaluation of the LEB and conventional LF immunoassays

A picture of the strip was taken adjusting the camera parameters of the smartphone (ISO 160, A 1/10, Manual focus). The picture was evaluated using Image J software following the procedure reported by our group [Alvarez-Diduk R. *et al.* Paper strip-embedded graphene quantum dots: a screening device with a smartphone readout. *Sci Rep* **7**(1), 976 (2017)].

### Results

### Electrophoresis performance evaluation

To test whether the LEB device is able to perform an electrophoresis different parameters as voltage, nitrocellulose membrane pore size and pH (Figure 4) were evaluated. The assay was carried out using CdSe@ZnS QDs functionalized with streptavidin (Ø 20 nm), CdTe QDs (Ø 5 nm) and G-QDs (Ø 2.5 nm); Emission and excitation spectra and TEM images of these nanomaterials are shown in Figure 10, 11 and 12, respectively.

The effect of the nitrocellulose membrane pore size on the migration speed of the QDs is shown in Figure 4A. It was evaluated by measuring the distance travelled (cm) in one minute. The field strength was fixed by using sodium borate buffer 10 mM at pH 8 and an operating voltage of 240 V. As expected the particle mobility depended on the size, shape and charge of the QDs. Three nitrocellulose membranes (HF240, HF180 and HF075) with different capillarity flow rates (240s/cm², 180s/cm² and 075s/cm² respectively) were used. Flow rate is directly proportional to the pore size, for instance, HF240 is the nitrocellulose membrane that has the lowest capillarity flow rate (240 s are required to reach one cm²) and therefore, the smallest pore size compared to the other two membranes (see figure 13). Pictures were taken before and after applying electrophoresis (t=0 and 1 minute, respectively). Finally, the relative distance travelled was measured using Image J software. As expected, the QDs moved faster when using a bigger pore size nitrocellulose membrane. The CdSe@ZnS QDs moved slower than CdTe and G-QDs mainly due to its bigger size.

Figure 4B shows the behavior of the voltage on the electrophoretic mobility at 100, 200 and 300v. A higher voltage causes a faster movement of the QDs being the CdTe QDs the fastest. All the QDs moved towards the negative electrode after applying the voltage, since they are positively charged at pH 8.

To evaluate the effect of the pH on the particle mobility and speed, the electrophoresis buffer was adjusted to pH 8, 9 and 10. G-QDs were used for this experiment since it is well known that their surface functional groups are deprotonated when the pH value increases. At pH 8, the G-QDs moved towards the negative electrode and at pH 9 and 10 towards the positive electrode (Figure 4 C, D). At pH 9 the G-QDs have enough negative surface charge to slightly move towards the positive electrode. The velocity of the G-QDs is higher at pH 8 and 10 than at pH 9 because at the latest pH the global sum of G-QDs charges is closer to neutrality; this is an evidence that the electrophoresis is running properly.

### Separation of QDs by electrophoresis

Then, it was tested whether the electrophoresis is carried out through the separation of different types of QDs (Figure 5). In this context, 20 nm CdSe QDs (25 nM in 10 mM PBS pH 7.4), 5 nm CdTe QDs (25 nM in 10 mM PBS pH 7,4) and 5 nm G-QDs (in 10 mM PBS pH 7.4) were used. The G-QDs were doped with nitrogen and sulphur following the procedure of Dong, *et al.*

It can be observed in Figure 5A, a mixture of 3 µL of N-S-doped G-QDs with 3 µL of CdSe QDs. Magenta fluorescent CdSe QDs move faster than the light blue N-S-doped G-QDs to the left side of the strip (negative electrode) until there is a complete separation of the two quantum dots. In this case, the doping of the G-QDs with nitrogen and sulphur has changed the surface charge, leading to a faster electrophoresis mobility of the CdSe QDs (see figure 14). In this way, it is proven that the charge factor contributes more on the electrophoresis mobility than the physical dimension of the QDs. The separation of CdTe QDs from CdSe QDs was also performed by incubating 3 µL of each for 1 minute at room temperature and drop-casting the mixture in the same conditions as before. It is observed in Figure 5B (upper panel) that the mixture remains in the centre of the strip before the battery is switched on. After running the electrophoresis for 10 minutes the orange CdTe QDs moved faster than the magenta CdSe QDs (Figure 5B, lower panel), as expected due to their differences in size and charge.

### Detection of H-IgG in human serum and comparison with traditional LFA

As the last step, the detection was tested using H-IgG in human serum as a proof-of-concept. Antibodies against H-IgG was deposited in the test line (TL) and antibodies against sheep IgG in the control line (CL). The CL is useful to confirm the successful migration of the reagents along the strip, meaning that the assay was successful.Two different types of labels CdSe QDs and gold nanoparticles (AuNp) were used. The former is the most common fluorescent label and the latest is used as colorimetric label.

A calibration curve was performed by spiking the H-IgG in IgG depleted serum (concentration from 0 to 1 µg/mL). IgG depleted serum was used because usually IgG is present and the amount of IgG in serum varies depending on the sample. Furthermore, it is important to clarify that although the serum is IgG depleted, it still contains the usual interfering matrix components e.g., albumin and globulins.

The detection of the H-IgG is based on the formation of the "immunosandwich" between the antibodies in the TL and the bioconjugated QDs. In order to achieve this, serial dilutions of H-IgG were mixed with the bioconjugated labels (QDs and AuNp). During the incubation, the H-IgG was recognized by the labelled antibodies and after running the electrophoresis, the complex H-IgG/QDs or H-IgG/AuNp migrates towards the detection zone, in which the TL and CL are fixed.

Several tests were carried out to validate the developed LEB device and the results were compared with those obtained with a conventional LFA (prior art) using different concentrations of H-IgG in 10 mM PBS pH 7.4 and serum. It is shown in Figure 6A from i to iv the results obtained; i and ii show the electrophoretic bioassay in serum using QDs and AuNp respectively, iii and iv show the LFA using QDs in PBS and serum respectively. During the evaluation of the LFA strips by naked eye, it is evident that the signal intensity is very high in TL and CL in a clean matrix (PBS), but the signal is masked when using a complex matrix (serum). While it was easy to follow an increase in the TL signal in PBS (Figure 6A iii), it was impossible to distinguish a positive sample from the blank sample in serum (Figure 6A iv). However, when the same assay is carried out with the LEB device in serum the signal intensity in TL and CL is high enough to be distinguished by naked eye (Figure 6A i). Similarly, after conducting the LEB with AuNPs as colorimetric labels, the signal in TL and CL wasn't masked by the complex matrix and the strip background remained clean and white, enhancing the signal to noise ratio (Figure 6A ii).

The strips were quantitatively evaluated using Image J measuring the signal intensity in TL (subtracting the strip background intensity). It can be noticed in Figure 6B (line with squares) the calibration curve for the lateral flow assay in PBS, corresponding to the strips observed in Figure 6A (iii). It is represented by the equation *P.L. Intensity* = *55.84 [H-IgG]* + *17.7* with a regression coefficient *r2* =*0.97* with a limit of detection (LOD) of 0.19 µg/mL (calculated by substituting in the equation the average signal of the blank (19.7 a.u.) plus three times its standard deviation (3.0 a.u.)). In the same way the calibration curves for the electrophoresis assay in serum using QDs (line with diamonds) and AuNP (line with triangles) are represented by the equation *P.L. Intensity* = *35.593 [H-IgG]* + *6.47 r2* =*0.9868* and *P.L. Intensity* = *7.396 [H-IgG]* + *0.188 r2* = *0.9699*, respectively, with an estimated LOD of 0.17 µg/mL for the former and 0.23 µg/mL for the latest. Although, the sensitivity is higher for the LFA in PBS compared with the electrophoresis assay using serum, it is important to remark that both LOD in serum and in PBS are comparable. Besides it was impossible to obtain a calibration curve with the LFA in serum.

### EXAMPLE 2

Alternative design of a device of LEB according to the present invention. Unlike the device of example 1, the electrodes are integrated in the paper strip so that no buffer storage tanks are needed, thus allowing a more compact and easy-to-use design that avoids the risk of accidental leakage.

These examples show that the inventors have designed a miniaturized Lateral-Electrophoretic Bioassay (LEB), a paper-based electrophoretic device that is portable and can be powered by a smartphone or a similar device. The inventors have demonstrated the ability of the LEB to separate QDs of different sizes and charges and have analysed the effect of voltage, paper porosity and pH on the electrophoretic mobility of the QDs.

Furthermore, the inventors have demonstrated the ability of the LEB as a diagnostic tool for the detection of H-IgG in human serum using QDs and AuNP as optical labels. The device is able to carry out the detection of the analyte by overcoming the limitation of high matrix effect in biosensors. The versatility of the LEB technology regarding the use of different nanomaterials and biorecognition elements allows its application in various fields, from novel nanomaterials characterization to point-of-care biomedical diagnostics.

## Claims

1. A strip test device comprising:
- means for supporting one or more paper strips for lateral flow assay, said paper strips comprising a sample application area (1) and a detection area (4) comprising capture elements; and
- means for producing an electrical potential difference in said one or more paper strips between the sample application area (1) and the detection area (4).

2. The device according to claim 1, wherein the means for producing an electrical potential difference in the one or more paper strips comprise:
- an electrical power source or a connector adapted to connect the device to an external electrical power source;
- at least a pair of electrodes of opposite charge connected to the power source or to the external power source connector, and being disposed spaced apart from each other so that one electrode or more electrodes are in fluid communication with one or more paper strips in a region located before the application sample area thereof and the opposite electrode or electrodes are in fluid communication with the one or more paper strips in a region located after the detection area thereof; and
- means for providing electrolytic solution so as to keep the electrodes and the paper strips in contact with electrolytic solution.

3. The device according to claim 2, wherein the means for providing an electrolytic solution to the electrodes and to the paper strips comprise reservoirs, wicks or the like.

4. The device according to any one of claims 2 or 3, wherein the connector adapted to connect to an external electrical power source comprises an adapter to increase the voltage of the external electrical power source.

5. The device according to any one of claims 2 or 3, wherein the connector adapted to connect to an external electrical power source comprises (i) an intermediate Joule thief inverter to increase the voltage of the external electrical power source and to change incoming direct current (DC) to alternating current (AC) and (ii) a rectifier to convert back alternating current (AC) to direct current (DC).

6. The device according to any one of the preceding claims, wherein the device is powered by a portable computing device.

7. The device according to any one of the preceding claims, wherein the device is configured to operate as a dipstick to be dipped into a sample to be tested or as a rigid plastic cassette provided with a sample well.

8. The device according to any one of the preceding claims, wherein the device comprises one or more leds pointing to the detection area (4) of the paper strips.

9. A method for the detection of an analyte in a sample with the device according to any one of the precedent claims, the method comprising:
- dispensing the sample onto the application area (1) of the one or more paper strips, wherein said sample either has been previously treated with conjugate outside the device or is treated with conjugate present on the device;
- producing an electrical potential difference across the one or more paper strips so that the sample starts migrating along the one or more paper strips by electrophoresis; and
- continuing the migration of the sample towards a detection area (4) of the one or more paper strips so that the presence of analyte may be determined by a detectable signal at the detection area (4).

10. The method according to claim 9, wherein a portable computing device is used as optical readout and/or as a power source.

11. Use of the device according to any one of claims 1 to 8 for the detection of an analyte in a sample.

12. A kit comprising the device according to any one of claims 1 to 8, instructions for use of the device and one or more paper strips.
